# EUROPÄISCHE PATENTSCHRIFT

(11) **EP 0 680 476 B1**
(45) Veröffentlichungstag und Bekanntmachung des Hinweises auf die Patenterteilung: **07.05.1997**
(21) Anmeldenummer: 94905045.4
(22) Anmeldetag: 12.01.1994
(51) Int. Cl.: C07D 307/92

(54) **VERFAHREN ZUR HERSTELLUNG VON NORAMBREINOLID**
PROCESS FOR PRODUCING NORAMBREINOLID
PROCEDE POUR LA FABRICATION DE NORAMBREINOLIDE

(30) Priorität: 21.01.1993 DE 4301555
(43) Veröffentlichungstag der Anmeldung: 08.11.1995
(73) Patentinhaber: Henkel Kommanditgesellschaft auf Aktien, 40191 Düsseldorf (DE)
(72) Erfinder: BOMHARD, Andreas, D-40591 Düsseldorf (DE); CASSEL, Jonathan, Foster City, CA 94404 (US); OLIVERO, Alan, Half Moon Bay, CA 94019 (US)
(86) Internationale Anmeldenummer: EP9400079
(87) Internationale Veröffentlichungsnummer: WO9417053

(56) Entgegenhaltungen:
- DD-A- 13 617
- Chemical Abstracts, Band 55, 1961, (Columbus, Ohio, US), GBnther Lucius et al: "Cyclization of homologous sesquiterpenic acids. III. The acid-catalyzed cyclization of homofarne- sylic acid", siehe Kolumne 4572 a-b, & Chem. Ber. 93, 2663-7 (1960)
- Chemical Abstracts, Band 114, Nr. 15, 15 April 1991, (Columbus, Ohio, US), Seite 816, Zusammenfassung 143747g; & JP,A,2258773, (Oritani Takayuki et al.) 19 Oktober 1990
- Patent Abstracts of Japan, Band 10, Nr 112(C-342); & JP,A,60239481 (HASEGAWA KORYO K.K. et al.), 1985-11-28

## Beschreibung

### Gebiet der Erfindung

Die Erfindung betrifft ein Verfahren zur Herstellung von Norambreinolid durch Cyclisierung von Homofarnesylsäure in Gegenwart von Methansulfonsäure.

### Stand der Technik

Ambroxan ist ein wertvoller Ambrariechstoff, der in einer Stoffwechselausscheidung des Pottwals enthalten ist. Die zunehmende Nachfrage nach Ambroxan hat bei begrenzten natürlichen Ressourcen in den letzten Jahren zur Entwicklung von Verfahren geführt, durch die Ambroxan aus preiswerten Rohstoffen synthetisch zugänglich ist.

Wegen der Schlüsselstellung des Ambroxans auf dem Gebiet der Riechstoffe besteht ein genereller Bedarf, verbesserte synthetische Zugangsmöglichkeiten zu entwickeln. Dieser Bedarf erstreckt sich insbesondere auf Verfahrensverbesserungen einzelner Teilschritte sowie die Entwicklung alternativer Zugangsmöglichkeiten zur Herstellung von wichtigen Zwischen- bzw. Vorprodukten des Ambroxans.

Wegen der begrenzten Verfügbarkeit natürlicher Rohstoffe, aus denen sich Ambroxan gewinnen läßt, spielen in jüngster Zeit sogenannte "synthetische Ambroxane" eine wichtige Rolle in der Riechstoffindustrie. Diese Produkte sind dadurch gekennzeichnet, daß sie neben Ambroxan auch dessen Epimere in nennenswerten Mengen enthalten (jeweils in racemischer Form); besonders zu erwähnen sind in diesem Zusammenhang 8-Epi- und 9-Epi-Ambroxan.

Da die einzelnen Epimeren unterschiedliche Geruchs-Charakteristika aufweisen, wird der Wert eines synthetischen Ambroxans durch seinen Gehalt an den einzelnen Epimeren bestimmt. Das geruchlich wertvollste Epimere ist dabei das (±)-Ambroxan [IUPAC-Name: (±)-(3a.alpha.,5a.beta.,9a.alpha.,-9b.beta.)-3a,6,6,9a-Tetramethyl-dodecahydro-naphtho[2,1-b]furan], gefolgt von (±)-9-Epi-Ambroxan. Das (±)-8-Epi-Ambroxan besitzt dagegen eine weniger erwünschte Geruchsnote.

Der Wert eines synthetischen Ambroxans als Riechstoff ist dementsprechend umso höher einzustufen, je höher insbesondere sein Gehalt an (±)-Ambroxan, aber auch an (±)-9-Epi-Ambroxan ist.

Ein besonderes attraktives Vorprodukt des Ambroxans ist das unter der Bezeichnung **Norambreinolid** bekannte Lacton, das in analoger Weise wie oben für das Ambroxan beschrieben in Form von Epimeren vorkommt. Der Wert des Norambreinolids als Vorstufe für Ambroxan bzw. ein synthetisches Ambroxan steht dabei in unmittelbarem Zusammenhang mit seinem Gehalt an den einzelnen Epimeren. Ein Norambreinolid ist umso wertvoller, je höher sein Gehalt an (±)-Sclareolid ist, das konfigurativ dem (±)-Ambroxan entspricht und dessen Vorstufe darstellt. Von Bedeutung ist ebenso das (±)-9-Epi-Sclareolid, während das (±)-8-Epi-Sclareolid unerwünscht ist.

Arbeiten zur Synthese von Norambreinolid aus gut verfügbaren Rohstoffen sind seit langem bekannt. Dabei kommt der Cyclisierung von Homofarnesylsäure eine besondere Bedeutung zu. Über die ersten Versuche zur sauren Cyclisierung von Homofarnesylsäure hat bereits G.Lucius in den 60-er Jahren berichtet; Lucius führte die Cyclisierung bei 40 °C in Gegenwart von Ameisensäure und Schwefelsäure als Katalysatoren durch (vergl. Chem. Ber. 1960, 2663). Er erhielt dabei in einer Gesamtausbeute von lediglich 14,7% ein Gemisch der stereoisomeren Lactone, das jedoch kein (±)-Sclareolid ("trans-anti-trans-Norambreinolid") enthielt.

G.Staiger und A.Macri beschreiben in der deutschen Patentanmeldung DE-OS 32 40 054 die Cyclisierung von Homofarnesylsäure mit Zinntetrachlorid bei -78 °C. Als Gesamtausbeute für Norambreinolid geben sie 91% an. Angaben zur Zusammensetzung des Gemisches und insbesondere des Gehaltes an den Norambreinolid-Epimeren (±)-Sclareolid und (±)-9-Epi-Sclareolid sind jedoch nicht dokumentiert. Darüber hinaus erscheinen sowohl der verwendete Cyclisierungskatalysator als auch die angewandte Temperatur im Hinblick auf eine Nutzung des Verfahrens im technischen Maßstab als sehr problematisch.

A.Saito et al. führten die Cyclisierung in Gegenwart von Bortrifluorid-Etherat bei -20 °C in Methylenchlorid als Lösungsmittel durch (vergl. Chem. Lett. 1984, 591 sowie JP 60/123 483). Die Handhabung von Bortrifluorid-Etherat ist jedoch wegen seiner Toxizität, der möglichen Freisetzung des hochentzündlichen Diethylethers während der Umsetzung und der Aufarbeitung sowie der problematischen Entsorgung der Rückstände mit besonderen Schwierigkeiten verbunden. Darüber hinaus haben die Autoren den Erfolg ihrer Methode lediglich im Maßstab von weniger als einem Gramm und mit hoch aufgereinigter Homofarnesylsäure dokumentiert.

Aus der japanischen Offenlegungsschrift JP 90/258 773 (Kuraray) ist die Cyclisierung von Homofarnesylsäure zu Norambreinolid in Gegenwart von Chlorsulfonsäure und in Nitromethan oder halogenierten Kohlenwasserstoffen als Lösungsmittel bei tiefen Temperaturen, insbesondere -30 °C bis -80 °C, bekannt. Konkret offenbart ist jedoch nur ein Beispiel, bei dem die Reaktion in 2-Nitropropan bei -70 °C mit einem 6-molaren Überschuß an Chlorsulfonsäure durchgeführt wurde. Als Ergebnis dieser Umsetzung wurde in nicht beschriebener Ausbeute (±)-9-Epi-Sclareolid erhalten. Das Verfahren ist jedoch wegen der Cancerogenität des Lösungsmittels, dem hohen Überschuß an Chlorsulfonsäure und der tiefen Reaktionstemperatur für eine technische Nutzung wenig attraktiv.

DD-A-13 617 beschreibt ein Verfahren zur Herstellung und Cyclisierung von Homofarnesylsäure und deren Estern. Dabei wird 4,8,12-Trimethyltridecatrien-2,7,11-säure in der Hitze mit Alkalilauge behandelt, die gebildete 4,8,12-Trimethyl-tridecatrien-3,7,11-säure durch partielle Veresterung als Ester abgetrennt, die nicht veresterte Ausgangssäure immer wieder der Alkalibehandlung zugeführt und aus dem Ester oder der daraus freigesetzten Säure in an sich bekannter Weise mit sauren Cyclisierungsmitteln carbobicyclische Lactone, Ester und Säuren gebildet.

Gemäß JP-A-60239481 wird (±)-Trans-β-Monocyclohomofarnesylsäure oder dessen Ester katalytisch mit Trifluoressigsäure umgesetzt. Die dabei unter Ringschluß entstehende Zwischenverbindung wird mit Natrium-bis-(2-methoxyethoxy)-aluminumhydrid reduziert, wobei der in der Zwischenverbindung vorhandene Lactonring unter Ausbildung einer Diol-Struktur geöffnet wird. Das Diol wird anschließend mit p-Toluolsulfonsäurechlorid in Gegenwart einer Base dehydrierend cyclisiert.

Die aus dem Stand der Technik bekannten Verfahren zur Herstellung von Norambreinolid aus Homofarnesylsäure vermögen insgesamt in verschiedenster Hinsicht nicht zu befriedigen, da sie jeweils mindestens einen der folgenden Nachteile aufweisen:
(1) Verwendung von Katalysatoren, die aufgrund ihrer Korrosivität und/oder Toxizität und/oder der Freisetzung leichtentziindlicher Stoffe (Ether aus Bortriflourid-Etherat) nur unter besonderen Sicherheitsvorkehrungen gehandhabt werden können;
(2) Verwendung von Lösungsmitteln, die für ein Verfahren im technischen Maßstab wenig geeignet sind;
(3) Verwendung von Katalysatoren und/oder Lösungsmitteln, die im Hinblick auf die Aufarbeitung des Reaktionsgemisches und/oder die Entsorgung der entstehenden Abfälle problematisch sind;
(4) Durchführung des Verfahrens bei extrem niedrigen Temperaturen, die technisch nur mit großem Aufwand realisiert werden können;
(5) Schlechte Ausbeuten an den erwünschten Norambreinolid-Epimeren (±)-Sclareolid und (±)-9-Epi-Sclareolid.

Darüber hinaus ist keinem Dokument des bekannten Standes der Technik zu entnehmen, ob die Cyclisierungsreaktion auch mit Homofarnesylsäure technischer Qualität, d.h. relativ unreiner Homofarnesylsäure, durchführbar ist.

### Beschreibung der Erfindung

Es bestand daher die Aufgabe, ein Verfahren zur Herstellung von Norambreinolid aus Homofarnesylsäure zu entwickeln, das die genannten Nachteile des Standes der Technik vermeidet und das insbesondere bei guten Ausbeuten an (±)-Sclareolid und (±)-9-Epi-Sclareolid auch im technischen Maßstab leicht durchführbar ist.

Diese Aufgabe wurde erfindungsgemäß gelöst durch ein Verfahren zur Herstellung von Norambreinolid aus Homofarnesylsäure in Gegenwart eines inerten organischen Lösungsmittels und eines sauren Cyclisierungskatalysators, wobei man als Cyclisierungskatalysator Methansulfonsäure einsetzt und die Cyclisierung bei Temperaturen im Bereich von -25 bis 0 °C durchführt.

In einer bevorzugten Ausführungsform der vorliegenden Erfindung wird Methansulfonsäure in der 1- bis 3-molaren Menge - bezogen auf Homofarnesylsäure - eingesetzt.

In einer weiteren bevorzugten Ausführungsform der vorliegenden Erfindung wird die Cyclisierung der Homofarnesylsäure im Temperaturbereich von -20 bis -5 °C durchgeführt.

Die Wahl des inerten organischen Lösungsmittels unterliegt an sich keinen besonderen Beschränkungen. Halogenalkane, z. B. Methylenchlorid, sind jedoch als Lösungsmittel bevorzugt.

Das erfindungsgemäße Verfahren wird üblicherweise derart durchgeführt, daß man das Lösungsmittel sowie die Methansulfonsäure in einem Reaktor, der möglichst mit einem Inertgas wie Stickstoff oder Argon gespült wurde, vorlegt und die zu cyclisierende Homofarnesylsäure anschließend so zudosiert, daß die Temperatur des Reaktionsgemisches 0 °C nicht übersteigt.

Die Auswahl von Methansulfonsäure als Cyclisierungskatalysator sowie die gleichzeitige Einhaltung der Reaktionstemperatur im Bereich von -25 bis 0 °C bewirkt in nicht vorhersehbarer und überraschender Weise die Bildung von Norambreinolid mit einem hohen Gehalt an den erwünschten Epimeren (±)-Sclareolid und (±)-9-Epi-Sclareolid. Dies ist besonders überraschend im Hinblick auf die erwähnte japanische Patentanmeldung JP 90/258 773, bei der die Cyclisierung mit Chlorsulfonsäure bewirkt wird. Der Fachmann hätte nämlich erwartet, daß unter den schonenden Bedingungen, die in dem Ausführungsbeispiel der JP 90/258 773 beschrieben sind (Reaktionstemperatur: -70 °C) das thermodynamisch am wenigsten stabile Epimere (±)-Sclareolid bevorzugt gebildet wird (vergl. dazu die Ausführungen von G.Lucius, loc. cit.). Stattdessen wird jedoch gemäß dem Verfahren der JP 90/258 773 kein (±)-Sclareolid gebildet, sondern das erst in zweiter Linie interessante (±)-9-Epi-Sclareolid.

Das erfindungsgemäße Verfahren hat darüber hinaus den Vorteil, daß Methansulfonsäure nach Neutralisation biologisch leicht abbaubar ist und außerdem in nur mäßigem Überschuß - bezogen auf Homofarnesylsäure - eingesetzt werden muß.

Ein weiterer Vorteil des erfindungsgemäßen Verfahrens besteht darin, daß Homofarnesylsäure in relativ unreiner Form, d.h. in der dem Fachmann geläufigen "technischen Qualität", eingesetzt werden kann. Dies ist für ein technisches Verfahren besonders attraktiv, da auf eine vorherige aufwendige Reinigung der Homofarnesylsäure verzichtet werden kann.

Die folgenden Beispielen dienen der Erläuterung der Erfindung und sind nicht einschränkend zu verstehen.

### Beispiele

### Verwendete Rohstoffe:

**Homofarnesylsäure** wurde in Form eines (technischen) 2:1 Gemisches seiner E,E- und Z,E-Isomeren eingesetzt. Dabei bedeutet "Z,E-Isomer" im Sinne der üblicherweise verwendeten Nomenklatur (3Z,7E)-4,8,12-Trimethyl-3,7,11-tridecantriensäure und entsprechend "E,E-Isomer" (3E,7E)-4,8,12-Trimethyl-3,7,11-tridecantriensäure.

### Beispiel 1 (gemäß der Erfindung)

In einem Reaktionsgefäß aus Edelstahl wurden 80 1 Methylenchlorid und 7,0 kg (73 mol) Methansulfonsäure vorgelegt. Der Reaktor wurde mit Stickstoff gespült und auf ca. -15 °C vorgekühlt. Unter ständigem Rühren wurden anschließend 10 kg 76%ige technische Homofarnesylsäure (30 mol) so zudosiert, daß die Temperatur des Reaktionsgemisches -10 °C nicht überstieg. Nach vollständiger Zugabe der Homofarnesylsäure wurde noch eine weitere Stunde bei ca. -10 °C gerührt. Unter externer Kühlung wurden anschließend 20 1 Wasser zugesetzt, wobei die Innentemperatur unterhalb von 5 °C gehalten wurde. Durch Zugabe von 10gew.-%iger Natronlauge wurde der pH-Wert der wäßrigen Phase anschließend auf ca. 4 eingestellt. Die organische Phase wurde abgetrennt, zweimal mit etwa 10 l Wasser gewaschen und eingedampft.

Das zurückbleibende braune Öl wurde in 25 l Methyl-tert.-Butylether gelöst und zweimal mit je 10 l 5gew.-%iger Natronlauge gewaschen. Die Wasserphasen dieser beiden Wäschen wurden vereinigt und mit 5 l Methyl-tert.-Butylether extrahiert. Anschließend wurden beide Etherphasen vereinigt und zweimal mit je 15 l 10gew.-%iger Natriumsulfatlösung und einmal mit 10 l Wasser gewaschen (pH-Wert des Waschwassers: 8). Der Ether wurde abdestilliert, wobei das Norambreinolid in Form eines orangeroten Öls in einer Menge von 7,3 kg anfiel. Beim Stehenlassen kristallisierte dieses Öl langsam durch.

Die gaschromatographische Analyse des Öls ergab einen Gehalt von 25 % (±)-Sclareolid sowie 21 % (±)-9-Epi-Sclareolid.

### Vergleichsbeispiel 1

Beispiel 1 wurde wiederholt, wobei jedoch anstelle der Methansulfonsäure 73 mol Chlorsulfonsäure eingesetzt wurden.

Das erhaltene Norambreinolid enthielt (±)-9-Epi-Sclareolid (Ausbeute: 19%) und (±)-8-Epi-Sclareolid (Ausbeute: 25%). Das wegen seiner geruchlichen Eigenschaften besonders präferierte (±)-Sclareolid wurde jedoch nicht gefunden.

## Patentansprüche

1. Verfahren zur Herstellung von Norambreinolid aus Homofarnesylsäure in Gegenwart eines inerten organischen Lösungsmittels und eines sauren Cyclisierungskatalysators, **dadurch gekennzeichnet,** daß man als Cyclisierungskatalysator Methansulfonsäure einsetzt und die Reaktion bei Temperaturen im Bereich von -25 bis 0 °C durchführt.

2. Verfahren nach Anspruch 1, wobei man Methansulfonsäure in der 1- bis 3-molaren Menge - bezogen auf Homofarnesylsäure - einsetzt.

3. Verfahren nach Anspruch 1 oder 2, wobei man die Reaktionstemperatur im Bereich von -20 bis -5 °C einstellt.

4. Verfahren nach einem der Ansprüche 1 bis 3, wobei man als inertes organisches Lösungsmittel ein Halogenalkan einsetzt.

## Claims

1. A process for the production of norambreinolide from homofarnesylic acid in the presence of an inert organic solvent and an acidic cyclization catalyst, characterized in that methanesulfonic acid is used as the cyclization catalyst and the reaction is carried out at temperatures of -25 to 0°C.

2. A process as claimed in claim 1, characterized in that methanesulfonic acid is used in 1 to 3 times the molar quantity, based on homofarnesylic acid.

3. A process as claimed in claim 1 or 2, characterized in that the reaction temperature is adjusted to a value of -20 to -5°C.

4. A process as claimed in any of claims 1 to 3, characterized in that a haloalkane is used as the inert organic solvent.

## Revendications

1. Procédé de fabrication de norambréinolide à partir d'acide homofarnésylique, en présence d'un solvant organique inerte et d'un catalyseur de cyclisation acide, **caractérisé en ce que** l'on utilise de l'acide méthanesulfonique comme catalyseur de cyclisation et on opère la réaction à des températures situées dans l'intervalle de -25 à 0 °C.

2. Procédé selon la revendication 1, dans lequel on met en oeuvre l'acide méthanesulfonique en quantité mono- à trimolaire par rapport à l'acide homofarnésylique.

3. Procédé selon la revendication 1 ou 2, dans lequel on ajuste la température réactionnelle dans l'intervalle de -20 à - 5 °C.

4. Procédé selon une des revendications 1 à 3, dans lequel on met en oeuvre un alcane halogéné comme solvant organique inerte.
